# EUROPÄISCHE PATENTSCHRIFT

(11) **EP 3 566 553 B1**
(45) Veröffentlichungstag und Bekanntmachung des Hinweises auf die Patenterteilung: **09.12.2020**
(21) Anmeldenummer: 17835582.2
(22) Anmeldetag: 20.12.2017
(51) Int. Cl.: H05H 1/24, A61L 2/14

(54) **PERMANENTE WUNDAUFLAGE MIT PLASMAELEKTRODE**
PERMANENT WOUND DRESSING WITH PLASMA ELECTRODE
PANSEMENT PERMANENT À ÉLECTRODE À PLASMA

(30) Priorität: 06.01.2017 DE 102017100192
(43) Veröffentlichungstag der Anmeldung: 13.11.2019
(73) Patentinhaber: Cinogy GmbH, 37115 Duderstadt (DE)
(72) Erfinder: HAHNL, Mirko, 37339 Berlingerode (DE); STORCK, Karl-Otto, 37115 Duderstadt (DE); TRUTWIG, Leonhard, 37115 Duderstadt (DE); WANDKE, Dirk, 37308 Heilbad Heiligenstadt (DE)
(74) Vertreter: Gramm, Lins & Partner Patent- und Rechtsanwälte PartGmbB
(86) Internationale Anmeldenummer: PCT/DE2017/101088
(87) Internationale Veröffentlichungsnummer: WO 2018/127255

(56) Entgegenhaltungen:
- EP-A1- 2 170 022
- WO-A1-2015/091070
- DE-A1-102014 013 716
- DE-A1-102014 220 488
- US-A1- 2015 343 231
- US-A1- 2016 271 411
- US-A1- 2016 331 989

## Beschreibung

Die Erfindung betrifft ein Behandlungsgerät für eine dielektrisch behinderte Plasmabehandlung einer Wund- oder Hautfläche, mit einer flexiblen flächigen Elektrodenanordnung mit wenigstens einer flächigen Elektrode und einer die wenigstens eine Elektrode zumindest teilweise einbettenden, Dielektrikums-schicht, die eine der Wund- oder Hautfläche zugewandte Auflageseite aufweist und die flächige Elektrode elektrisch von der Wund- oder Hautfläche so ab-schirmt, dass nur ein dielektrisch behinderter Stromfluss von der Elektrode zur Wund- oder Hautfläche möglich ist, und mit einem ein separates Gehäuse auf-weisenden Steuergerät, über das die Elektrode mit einer Betriebsspannung verbindbar ist.

Ein derartiges Behandlungsgerät ist durch DE 10 2014 013 716 A1 bekannt. Die flexible flächige Elektrodenanordnung ist dabei so ausgebildet, dass sie mit ihrer Auflageseite unmittelbar auf einer Wundfläche aufliegen kann. Hierzu ist das Dielektrikum selbst aus einem wundverträglichen Material gebildet oder das Dielektrikum auf der Auflageseite mit einer wundheilenden Schicht versehen, die als übliche Mull-Zelluloseschicht oder auch als eine Schicht aus einer festen, offen-porigen Matrix aus einem pflegenden oder heilungsfördernden Material sein kann. Die Schicht kann dabei unmittelbar auf das Dielektrikum aufgewachsen sein. Aufgrund der Offenporigkeit der Schicht kann sich das gewünschte Plasma in dem Material der Matrix ausbilden. Das Dielektrikum selbst kann auf der Auflageseite mit einer Struktur versehen sein, die beispielsweise aus Noppen bestehen und auf der Wundfläche aufliegen kann und zwischen sich Lufträume aus-bildet, in denen das Plasma aufgrund der elektrischen Ansteuerung der Elektro-de entstehen kann. Bei dem bekannten Behandlungsgerät kann die flächige Elektrodenanordnung als ein einstückiges Auflagestück ausgebildet sein und einen zungenförmigen Ansatz aufweisen, mit dem das Auflagestück in einen Aufnahmeschlitz des als Griff ausgebildeten Gehäuses des Steuergeräts einsteckbar sein kann. Im eingesteckten Zustand kann die Elektrode mit der im Steuergerät gebildeten Betriebsspannung in Form von für die Plasmabildung geeigneten Hochspannungssignalen kontaktiert werden. Sowohl die Dielektrikums-schicht als auch die flächige Elektrode können dabei mit Durchgangsöffnungen versehen sein, die sowohl für eine Absaugung von Fluid aus dem Wundbereich als auch für das Einblasen von für die Wundheilung fördernden Gasen geeignet sind.

Das bekannte Behandlungsgerät ist dafür geeignet, mit einer kurzzeitigen Plasmabehandlung die Wundfläche zu desinfizieren, sodass danach eine übliche Wundabdeckung, ggf. getränkt mit einem die Wundheilung fördernden Arznei-mittel wieder für einige Stunden aufgelegt werden kann. Danach wird die Wund-auflage wieder abgenommen und eine erneute Plasmabehandlung mit dem Behandlungsgerät durchgeführt, um eine Anreicherung von Mikroorganismen in der Wundfläche bzw. in dem Wundsekret zu vermeiden. Neben der Desinfektionswirkung wird durch die Plasmabehandlung die Mikrozirkulation in der Haut angeregt.

Es ist daher auch bekannt, eine nicht durch eine Wunde gestörte Hautfläche einer Plasmabehandlung zu unterziehen, um neben der Desinfektionswirkung insbesondere eine heilende oder straffende Wirkung durch die erhöhte Mikrozirkulation zu erreichen.

Ein Behandlungsgerät für eine langandauernde Behandlung ist in US 2016/0031989 A1 beschrieben. Das Gerät weist ein flexibles flächiges Auflagestück auf, das eine Elektrodenanordnung mit einem Dielektrikum beinhaltet. Die Anordnung ist auf einem Polymerfilm aufgebracht, der für eine gute Durchlüftbarkeit mit zahlreichen Durchgangsöffnungen versehen ist. Die Auflageseite der Elektrodenordnung, die zur Anlage an der Haut einer Person bestimmt ist, ist mit einer Mullabdeckung versehen. Das Auflagestück ist mit einem Steuergerät verbindbar, in dem die Ansteuerungssignale für die Elektrodenanordnung generiert werden. Die Übertragung erfolgt über ein Hochspannungskabel mit einer Steckverbindung, für die die Elektrodenanordnung eine eingearbeitete Steuerbuchse aufweist. In einer Variante der Anordnung kann über die Steckverbindung an das Auflagestück eine flächige Tasche angesteckt werden, in der sich eine Batterieanordnung und ein Inverter für die Generierung der Steuersignale für die Elektrodenanordnung befindet. Als Anwendung für diese bekannte Anordnung sind ästhetische Behandlungen zur Entfernung von Falten und Wiederherstellung der Hautelastizität sowie Behandlungen von chronischen Hauterkrankungen, wie beispielsweise Fußpilz, beschrieben. Mit der Plasmabehandlung werden die Hautbereiche desinfiziert und aktiviert, sodass betroffene Hautbereiche, beispielsweise Verbrennungen, wiederhergestellt werden können, Haarausfall verhindert und Haarwuchs gefördert wird.

US 2016/0271411 A1 beschreibt ein Plasmabehandlungsgerät, das ebenfalls aus einem Steuergerät und einem auf ein Körperteil aufzubringenden Plasmaapplikator besteht. Das Steuergerät weist dabei eine Pumpe auf, mit der über eine Verbindungsleitung Gas oder Luft von dem Behandlungsraum zwischen Plasmaapplikator und Hautoberfläche absaugbar ist. Die Verbindungsleitung ist hierfür mit dem Plasmaapplikator lösbar verbindbar und bewirkt über eine Öffnung in dem scheibenförmigen Plasmaapplikator die Kommunikationsverbindung mit dem Absaugraum. Das Steuergerät kann mit Batterien versorgt werden, sodass es vom Patienten, beispielsweise für einen Spaziergang, getragen werden kann. Eine Befestigung des Steuergeräts am Körper ist nicht vorgesehen.

Der vorliegenden Erfindung liegt die Aufgabe zugrunde, ein Behandlungsgerät der genannten Art so auszubilden, dass eine verbesserte Behandlung einer Wund- oder Hautfläche möglich ist.

Zur Lösung dieser Aufgabe ist erfindungsgemäß ein Behandlungsgerät der eingangs erwähnten Art dadurch gekennzeichnet, dass die Anordnung aus Elektrode und Dielektrikumsschicht im Bereich der Durchgangsöffnungen mit einer luftdichten Abdeckung, die die Flexibilität der Elektrodenanordnung nicht beeinträchtigt, gasdicht abgedichtet überdeckt ist und so einen mit den Durchgangsöffnungen kommunizierenden Fluidraum begrenzt, dass an die Abdeckung eine Leitung angeschlossen ist und dass das Steuergerät eine Pumpe enthält, die über die Leitung mit dem Fluidraum verbindbar ist.

Das erfindungsgemäße Behandlungsgerät ist somit dafür ausgelegt, mit der Elektrodenanordnung aus Elektrode und Dielektrikumsschicht dauerhaft auf die Wund- oder Hautfläche aufgelegt zu werden, also nicht nur für die Zeit der Behandlung der Wund- oder Hautfläche mit einem Plasma zum Zwecke der Abtötung von Keimen und/oder Anregung der Mikrozirkulation im Gewebe. Es entfällt somit der bisher übliche Wechsel zwischen einer herkömmlichen Wundauflage und der Auflage einer Elektrodenanordnung zur Durchführung einer desinfizierenden Plasmabehandlung der Wundfläche. Damit die Anordnung aus Elektrode und Dielektrikumsschicht als in diesem Sinne ununterbrochene Auflage auf einer Wund- oder Hautfläche geeignet ist, ist die Anordnung so ausgebildet, dass eine Ansammlung von Wundsekret auf der Auflageseite durch die Durchgangsöffnungen abführbar ist. Dies wird dadurch unterstützt, dass auf der Oberseite der Dielektrikumsschicht, die der Auflageseite gegenüberliegt, aufsaugendes Material angeordnet wird, mit dem Wundsekret von der Auflageseite der Anordnung durch Durchgangsöffnungen transportiert wird, um an der Oberseite der Dielektrikumsschicht zu verbleiben. Der Abtransport von Wundsekret von der Wundfläche wird durch eine Unterdruckausbildung mittels einer Saugpumpe in an sich bekannter Weise gefördert. Für die Behandlung einer nicht mit einer Wundfläche versehenen Hautfläche können die Durchgangsöffnungen ggf. zur Zuführung einer heilenden Substanz verwendet werden.

Damit das erfindungsgemäße Behandlungsgerät für eine dauerhafte Wund- oder Hauptauflage geeignet ist, wird das Steuergerät in unmittelbarer Nähe der als Wund- oder Hautauflage dienenden Anordnung aus Elektrode und Dielektrikumsschicht an dem die Wund- oder Hautfläche aufweisenden Körper des Lebewesens befestigt. Auf diese Weise ist es dem Träger des Behandlungs-geräts möglich, während der Wundheilung oder der Hautbehandlung mobil zu bleiben und die Bewegungsfreiheit wie mit einem herkömmlichen Wundverband, der die Möglichkeit einer Plasmabehandlung nicht bietet, zu haben. Dabei ist das Steuergerät klein und leicht ausgebildet und wird direkt am Körper befestigt, beispielsweise durch eine Befestigungsschlaufe, die beispielsweise um den Hals gelegt wird und/oder mit Hilfe eines Saugnapfes, mit dem eine Fixierung auf der Haut nach Art von EKG-Elektroden möglich ist. Selbstverständlich ist es auch möglich, das Steuergerät auf die Haut mit üblichen Pflasterklebstoffen aufzukleben. Dabei kann die Klebverbindung allein oder in Verbindung mit einer mechanischen Befestigung vorgesehen werden. Welche Art der Befestigung im Einzelfall sinnvoll ist, entscheidet sich nach der Lage der Wund- oder Hautfläche und den in der Umgebung der Wund- oder Hautfläche bestehenden Befestigungsmöglichkeiten. Angestrebt wird dabei jeweils, eine kurze Strecke zwischen der Auflageanordnung aus Elektrode und Dielektrikums-schicht einerseits und dem Steuergerät andererseits, insbesondere wenn über diese Verbindung ein Hochspannungssignal (Spitzenspannung von einigen kV) übertragen wird.

Zur Ermöglichung einer wirkungsvollen Absaugung ist in der vorliegenden Erfindung vorgesehen, dass die Dielektrikumsschicht mit Durchgangsöffnungen versehen ist und die Anordnung aus Elektrode und Dielektrikumsschicht im Bereich der Durchgangsöffnungen mit einer luftdichten Abdeckung gasdicht abgedichtet überdeckt ist und so einen mit den Durchgangslöchern kommunizierenden Fluidraum begrenzt, dass an die Abdeckung eine Leitung angeschlossen ist. Das Steuergerät enthält eine Pumpe, die über die Leitung mit dem Fluidraum verbindbar ist. In der vorliegenden Erfindung weist das Behandlungsgerät selbst die Pumpe auf, mit der beispielsweise eine Absaugung von Wundsekret, erfolgen kann, ohne dass dadurch die Handhab-barkeit des Behandlungsgeräts und die Beweglichkeit des Patienten beeinträchtigt werden.

Die abgesaugte Flüssigkeit kann in dem Absaugraum oberhalb der Dielektrikumsschicht angesammelt werden, wenn der Absaugraum zumindest teilweise mit einem flüssigkeitsabsorbierenden Material gefüllt ist.

In einer anderen Ausführungsform ist in dem Steuergerät der Pumpe im Strömungsweg ein Behälter zur Aufnahme von durch die Pumpe transportierten Fluid vorgeschaltet. Dadurch ist es möglich, auch Wundflächen zu behandeln, die große Mengen von Wundsekret absondern. Der Behälter ist vorzugsweise aus-wechselbar mit der Pumpe und/oder dem Gehäuse verbunden. Dabei ist es vor-teilhaft, wenn sich in dem Behälter im Strömungsweg zur Pumpe ein gasdurchlässiger, flüssigkeitszurückhaltender Filter befindet. Dadurch wird sichergestellt, dass keine abgesaugte Flüssigkeit in die Pumpe gerät, sondern in dem Behälter zurückgehalten wird. Wenn der Behälter austauschbar befestigt ist, kann das Steuergerät unproblematisch für einen weiteren Einsatzfall verwendet werden. Da die Komponenten des Steuergeräts billige Massenkomponenten sind, kann es für bestimmte Anwendungsfälle zweckmäßig sein, von einer Sterilisierung des Steuergeräts nach einem Einsatz abzusehen und das Steuergerät zusammen mit dem durch Elektrode und Dielektrikumsschicht gebildeten Auflagestück zu entsorgen.

Die beschriebene Anordnung mit den Durchgangsöffnungen und einer luftdichten Abdeckung eignet sich nicht nur zur Durchführung einer Absaugung, sondern auch zur Zuführung einer heilenden oder hautpflegenden Substanz in den Bereich der Hautfläche, in dem die Plasmabehandlung stattfindet. Hierdurch kann die Plasmabehandlung ggfs. wirkungsvoll unterstützt werden, weil durch die Plasmabehandlung die Aufnahmefähigkeit der Haut für zugeführte heilende oder pflegende Substanzen deutlich erhöht wird. Die Zuführung der Substanz kann dabei automatisiert erfolgen, wenn das Steuergerät hierfür eine Zuführ-pumpe aufweist, deren Ein- und Ausschaltung durch einen Mikrocontroller, vor-zugsweise in Koordination mit Perioden der Plasmabehandlung, gesteuert wird.

Für das Behandlungsgerät kann es vorteilhaft sein, wenn die Anordnung aus Elektrode und Dielektrikumsschicht ein zungenförmiges Anschlussstück ausbildet, das als schmaler Steg in einen Aufnahmeschlitz des Steuergeräts zur Herstellung einer elektrischen Verbindung zwischen der Betriebsspannung und der Elektrode einschiebbar und festlegbar ist. Auf diese Weise ist eine direkte Kontaktierung der Elektrode möglich, ohne dass hierfür ein Verbindungskabel benötigt wird. Die Verbindung zwischen der Anordnung und dem Steuergerät kann mittels einer Bedientaste lösbar ausgebildet sein.

In einer Ausführungsform des Behandlungsgeräts sind die wenigstens eine Elektrode und die Dielektrikumsschicht in einem einstückigen Auflagestück enthalten. Das Auflagestück ist erfindungsgemäß als dauerhafte Wund- oder Haut-auflage ausgebildet.

In einer Ausführungsform des Steuergeräts ist die im Steuergerät der Elektrode zugeführte Betriebsspannung durch Hochspannungssignale gebildet, mit denen die dielektrisch behinderte Plasmaentladung auf der Anlageseite der Dielektrikumsschicht entsteht, wobei der Körper im Bereich der Wund- oder Hautfläche als Gegenelektrode fungiert

In einer anderen Ausführungsform ist die im Steuergerät der Elektrode zugeführte Betriebsspannung eine Wechselspannung, die noch keine Hochspannung darstellt, sondern die über eine Hochspannungsstufe auf die Elektrode gelangt, wobei die Hochspannungsstufe auf dem Auflagestück angeordnet ist oder in das Auflagestück integriert ist. Ein Vorteil dieser Anordnung besteht darin, dass die Hochspannung unmittelbar an der Elektrode generiert werden kann und daher nicht über ein Verbindungsstück vom Steuergerät auf die Elektrode geleitet wer-den muss.

Die Vorteile des erfindungsgemäßen Behandlungsgeräts werden noch dadurch verstärkt, dass das Steuergerät eine Batteriespannungsversorgung aufweist, deren Ausgangsgleichspannung in dem Steuergerät mit einer Wandlerstufe in Wechselspannungssignale gewandelt wird. Die Batteriespannungsversorgung kann dabei mit üblichen Einmalbatterien, aber auch mit wiederaufladbaren Batterien (Akkumulatoren) gebildet sein.

Die Erfindung soll im Folgenden anhand von in der Zeichnung dargestellten Ausführungsbeispielen näher beschrieben werden. Es zeigen:
- Figur 1: eine schematische Draufsicht auf eine erste Ausführungsform eines Behandlungsgeräts mit einer Absaugvorrichtung;
- Figur 2: eine Seitenansicht des Behandlungsgeräts gemäß Figur 1;
- Figur 3: eine schematische Darstellung des Aufbaus des Steuergeräts der ersten Ausführungsform;
- Figur 4: eine schematische Draufsicht auf eine zweite Ausführungsform eines Behandlungsgeräts;
- Figur 5: eine Seitenansicht der Ausführungsform gemäß Figur 4;
- Figur 6: eine schematische Darstellung des Aufbaus des Steuergeräts gemäß der zweiten Ausführungsform;
- Figur 7: eine Draufsicht auf ein Auflagestück für die erste und zweite Ausführungsform;
- Figur 8: ein Hochschnitt durch das Auflagestück;
- Figur 9: eine Ansicht des Auflagestücks von der Auflageseite (von unten);
- Figur 10: eine Ansicht von unten auf eine Auflageseite einer Elektrodenanordnung mit einer einzigen flächigen Elektrode, wobei in die Dielektrikumsschicht eine Hochspannungsstufe integriert ist;
- Figur 11: einen Hochschnitt durch die Anordnung gemäß Figur 10;
- Figur 12: eine Ansicht auf die Auflageseite einer Elektrodenanordnung mit zwei Elektroden, die beide mit gegenphasigen Hochspannungssignalen versorgt werden, die in einer in das Auflagestück integriere Hoch-spannungsstufe erzeugt werden;
- Figur 13: einen Hochschnitt entlang der Linie A-A in Figur 12;
- Figur 14: eine Draufsicht auf eine dritte Ausführungsform des Behandlungsgeräts, bei der keine Absaugung mit einer Pumpe stattfindet;
- Figur 15: eine Seitenansicht der Ausführungsform gemäß Figur 14;
- Figur 16: eine schematische Darstellung des Aufbaus des Steuergeräts für die dritte Ausführungsform.

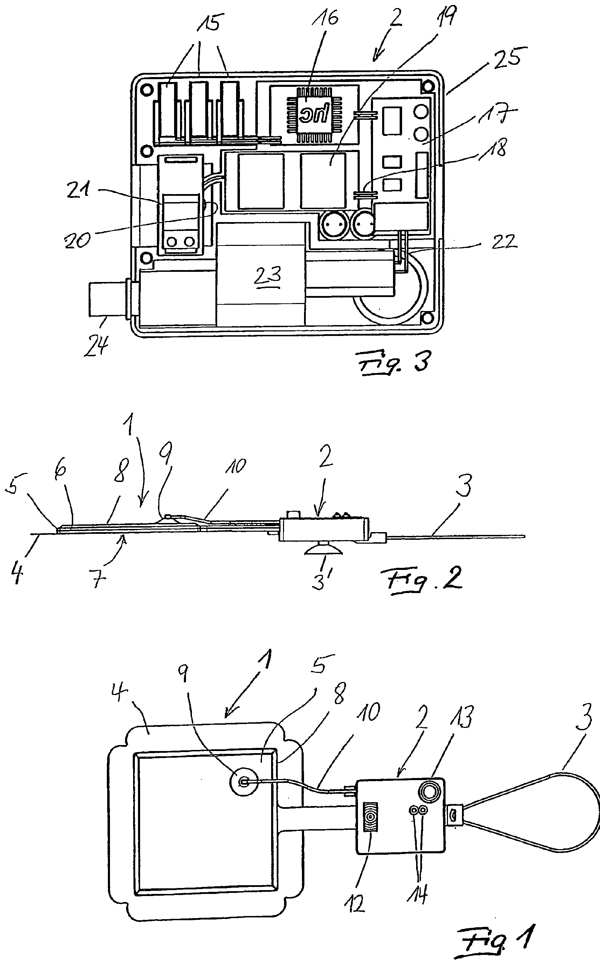

Die in den Figuren 1 bis 3 dargestellte erste Ausführungsform lässt ein Auflage-stück 1 erkennen, das unmittelbar mit einem Steuergerät 2 verbunden ist. Das Steuergerät 2 ist mit einer Befestigungsvorrichtung 3 in Form einer Befestigungsschlaufe versehen, um das Steuergerät 2 am Körper eines Patienten anzubringen.

Das Auflagestück 1 besteht aus einem rahmenförmigen Randstück 4, das an einer Unterseite mit einem Haftkleber versehen ist und zur haftenden Auflage auf der Haut eines Patienten, ggf. um eine Wundfläche herum, vorgesehen ist. Das Randstück 4 überragt allseitig eine Dielektrikumsschicht 5, die flächig und flexibel ausgebildet ist und eine flächige und flexible Elektrode 6 einbettet und allseitig gegen Berührung abschirmt. Die Dielektrikumsschicht 5 verhindert, dass von der Elektrode 6 zur als Gegenelektrode fungierenden Haut des Patienten ein direkter galvanischer Strom fließt, wenn die Dielektrikumsschicht mit ihrer Unter-seite (Auflageseite 7) auf der Haut des Patienten im Bereich einer Wund- oder Hautfläche aufliegt. Auf der Oberseite der Dielektrikumsschicht 5, also auf der der Auflageseite gegenüberliegenden Seite ist die Dielektrikumsschicht 5 mit einer gasdichten Abdeckung 8 luftdicht abgedeckt. Die Abdeckung beeinträchtigt die Flexibilität des Auflagestücks 1 nicht und ist beispielsweise in Form einer Folie ausgebildet. Auf eine Öffnung der Abdeckung 8 ist ein Absaugnapf 9 angeordnet, an den eine Schlauchleitung 10 angeschlossen ist, die zu dem Steuergerät 2 führt.

Die Elektrode 6 und die Dielektrikumsschicht 5 bilden gemeinsam einen zungen-förmigen Ansatz 11 aus, mit dem sich die von der Dielektrikumsschicht 5 umhüllte Elektrode 6 in das Steuergerät 2 hineinerstreckt. Das Steuergerät 2 ist mit einer Taste 12 versehen, mit der die Verbindung des zungenförmigen Ansatzes 11 mit dem Steuergerät 2 lösbar ist. Figur 1 lässt ferner eine Ein-/Aus-Taste 13 sowie zwei Anzeigeleuchten 14 zur Anzeige von Betriebszuständen des Steuer-geräts 2 erkennen.

Figur 2 verdeutlicht ferner, dass das Steuergerät 2 noch mittels einer weiteren Befestigungsvorrichtung 3', hier in Form eines Saugnapfes, am Körper des Patienten festlegbar ist. Die Befestigungsvorrichtungen 3 und 3' wirken somit zur Fixierung des Steuergeräts 2 am Körper des Patienten zusammen. Das in Figur 3 dargestellte Steuergerät 2 ist als kabelloses Gerät dargestellt und weist drei Batterien 15 auf, mit denen eine Batteriespannungsversorgung hergestellt wird. Die Versorgungsspannung durch die Batterien 15 gelangt auf einen Mikrocontroller 16, der den Ablauf des Steuergeräts 2 steuert. Der Mikrocontroller 16 steuert eine Wechselrichterstufe 17, mit der in an sich bekannter Weise aus der Gleichspannung der Batterien 15 eine Wechselspannung mit einer erhöhten Scheitelspannung von beispielsweise 250 V erzeugt wird. Die Wechselrichterstufe 17 speist über einen ersten Ausgang 18 eine Hochspannungsstufe 19, in der Hochspannungsimpulse entgegengesetzter Polarität von einigen 10 kV erzeugt werden. Dies geschieht in an sich bekannter Weise mit Hilfe von (nicht dargestellten) Zündstrecken, die bei Überschreiten einer Schwellspannung zünden und einen Stromimpuls durch eine Primärspule verursachen. Eine mit einem geeigneten Übertragerverhältnis gewickelte Sekundärspule lässt an ihrem Ausgang einen Hochspannungsimpuls entstehen, der auf einen Kontakt 20 einer Kontakteinrichtung 21 geleitet wird. Die Kontakteinrichtung 21 kann mit der Taste 12 verriegelt und entriegelt werden.

Ein zweiter Ausgang 22 der Wechselrichterstufe 17 speist eine Ansaugpumpe 23, die mit einem Schlauchstutzen 24 aus einem das Steuergerät 2 umgebenden Gehäuse 25 herausragt und die Verbindung mit der Schlauchleitung 10 ermöglicht.

Die Einschaltung und Ausschaltung der Hochspannungsversorgung durch die Hochspannungsstufe 19 kann zweckmäßigerweise durch ein im Mikrocontroller 16 abgelegtes Programm gesteuert werden. Beispielsweise kann die Plasmabehandlung über eine Zeitdauer von 1 bis 2 Minuten erfolgen, woran sich jeweils eine Pause von mehreren Stunden anschließen kann. Ferner kann die Absaugung durch die Absaugpumpe 23 in geeigneter Weise periodisch gesteuert wer-den, beispielsweise jeweils für einige Sekunden in Zeitabständen zwischen 10 und 60 Minuten bei akut nässenden Wunden erfolgen.

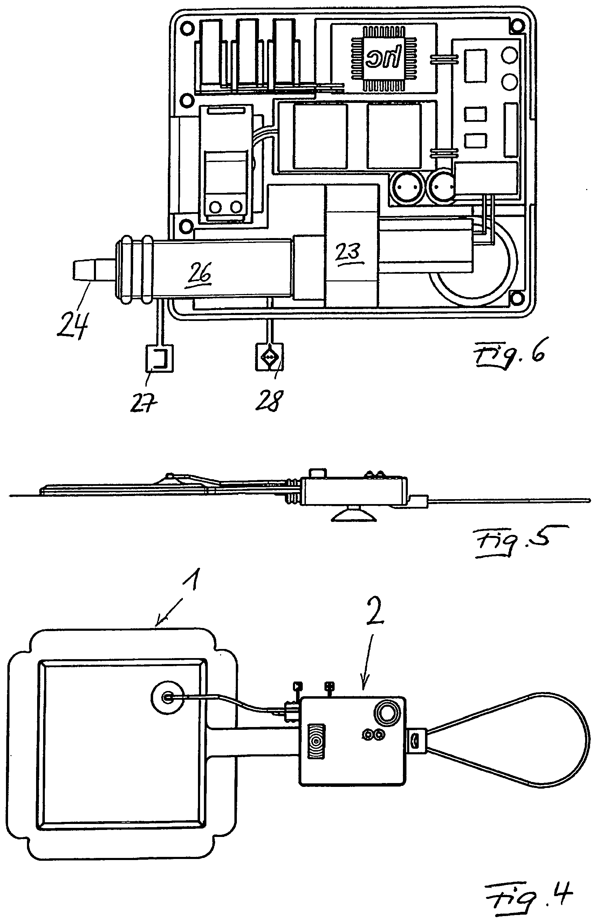

Bei der in den Figuren 4 bis 6 dargestellten Ausführungsform ist ein praktisch gleicher Aufbau des Auflagestücks 1 und des Steuergeräts 2 realisiert. Der einzige Unterschied besteht darin, dass die Ansaugpumpe 23 einen angesetzten Behälter 26 aufweist, der eine Aufnahmekammer (verdeutlicht durch Symbol 27) und einen Filter (verdeutlicht durch Symbol 28) aufweist. In der Aufnahmekammer 27 des Behälters 26 wird abgesaugte Flüssigkeit gesammelt. Der Filter 28 verhindert, dass die abgesaugte Flüssigkeit in die Ansaugpumpe 23 gerät. Nach Beendigung der Behandlung kann der Behälter 26 von der Pumpe 23 gelöst und mit der abgesaugten Flüssigkeit entsorgt werden. Figur 6 lässt erkennen, dass der Behälter 26 zwischen der Pumpe 23 und dem Anschlussstutzen 24 angeordnet ist.

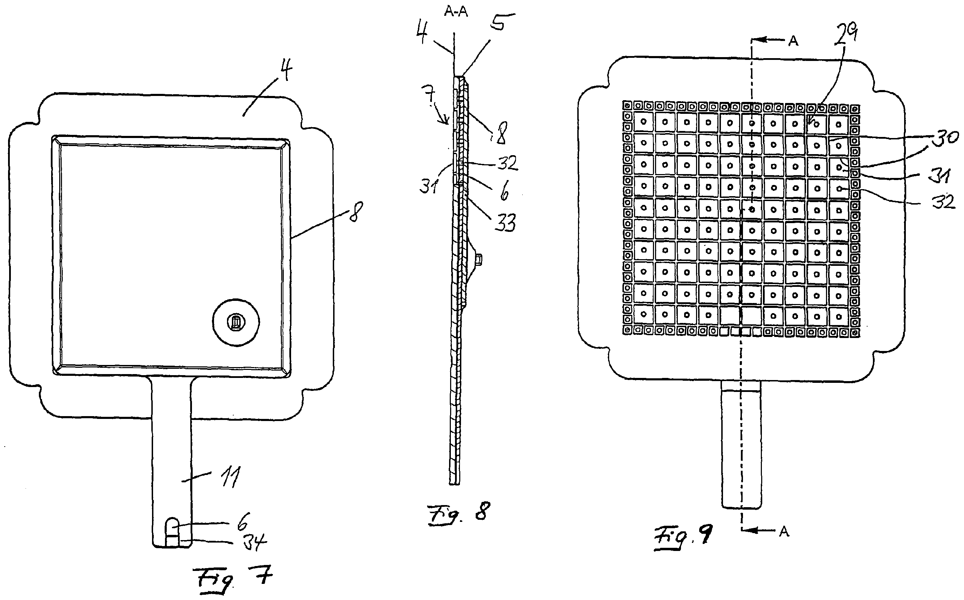

Die Figuren 7 bis 9 verdeutlichen in vergrößerter Darstellung den Aufbau des Auflagestücks 1. Insbesondere ist erkennbar, dass die Dielektrikumsschicht 5 an der Auflageseite 7 eine gitterförmige Struktur 29 ausbildet, die durch sich kreuzende, senkrecht zueinander stehende Stege 30 gleicher Höhe entsteht. Dadurch werden zur Auflageseite 7 hin offene Kammern 31 gebildet, die an ihrer Oberseite durch eine durchgehende Lage der Dielektrikumsschicht 5 von der in die Dielektrikumsschicht 5 eingebetteten Elektrode 6 abgeschirmt sind. Die Form der Kammern 31 kann auch rautenförmig, wabenförmig oder auch rund ausgebildet sein. Die Dielektrikumsschicht weist mittig in den Kammern 31 jeweils eine Durchgangsöffnung 32 auf, die in einen Absaugraum 33 münden, der durch die Abdeckung 8 einerseits und die Dielektrikumsschicht 5 andererseits begrenzt ist. Der Absaugraum 33 kann mit einem saugfähigen Material ausgefüllt sein, das die Luft-Absaugströmung durch die Ansaugpumpe 23 durchlässt, jedoch die ab-gesaugte Flüssigkeit aufhält. Die Füllung des Absaugraums 33 mit einem flüssigkeitsabsorbierenden Material ist insbesondere dann vorteilhaft, wenn gemäß der ersten Ausführungsform die Absaugpumpe 23 ohne vorgeschalteten Behälter 26 verwendet wird.

Die Schnittdarstellung der Figur 8 lässt erkennen, dass auch die Elektrode 6 Durchgangsöffnungen aufweist, die mit den Durchgangsöffnungen 32 fluchten, jedoch einen größeren Durchmesser aufweisen, sodass die Dielektrikumsschicht 5 durchgehend die Wandung der Durchgangsöffnungen 32 bildet, sodass ein direkter Kontakt von Flüssigkeit mit der die Hochspannung führenden Elektrode 6 nicht erfolgen kann. Die in den Durchgangsöffnungen 32 befindliche Stärke der Dielektrikumsschicht 5 ist so groß, dass Spannungsdurchschläge oder Mikroentladungen sicher vermieden werden.

Die Figuren 7 und 9 lassen erkennen, dass sich die Abdeckung 8 über den gesamten Bereich der Elektrode 6 und der Dielektrikumsschicht 5 erstreckt, in dem sich die Durchgangsöffnungen 32 befinden. Somit kommuniziert der Absaugraum 33 mit den Durchgangsöffnungen 32 dichtet aber im Übrigen die Dielektrikumsschicht 5 im Bereich des Absaugraums 33 gasdicht ab.

Der in Figur 7 erkennbare zungenförmige Ansatz 11 enthält einen streifenförmigen Ansatz der Elektrode 6, der vollständig von der Dielektrikumsschicht 5 um-schlossen ist, mit Ausnahme der in Figur 7 erkennbaren Ausnehmung 34, durch die ein kleines Stück der Elektrode 6 zur Oberseite hin freiliegt. Der zungenförmige Ansatz 11 wird in den entsprechenden Aufnahmeschlitz des Steuergeräts 2 eingeschoben, sodass mit Abstand von dem Schlitz innerhalb des Gehäuses 25 des Steuergeräts die Kontaktierung mit dem Kontakt 20 erfolgen kann.

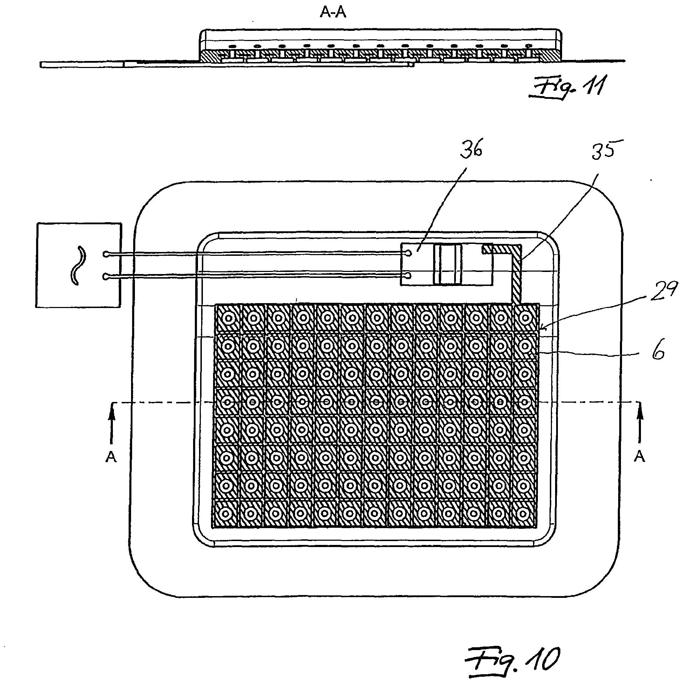

Die Figuren 10 und 11 lassen das Auflagestück 1 von der Auflageseite her er-kennen, wo die Dielektrikumsschicht die Gitterstruktur 29 aufweist. Im Bereich der Gitterstruktur 29 erstreckt sich auch die Ausdehnung der Elektrode 6, die in diesem Ausführungsbeispiel mit einem flächigen Ansatz 35 mit dem Ausgang einer Hochspannungsstufe 36 verbunden ist, die sich in dieser Ausführungsform innerhalb der Dielektrikumsschicht 5 im Auflagestück 1 befindet. Demgemäß wird das Auflagestück 1 gemäß Figur 10 lediglich mit einer Wechselspannung versorgt, wie sie beispielsweise an den Ausgängen 18, 22 der Wechselrichterstufe 17 in den Ausführungsformen der Figuren 1 bis 6 ansteht.

Die Hochspannungsstufe 36 ist vorzugsweise in das Material der Dielektrikumsschicht 5 integriert, das hierzu an dem Rand, in dem sich die Hochspannungs-stufe 36 befindet, mit einer vergrößerten Dicke ausgebildet sein kann, wie dies die Schnittdarstellung der Figur 11 verdeutlicht, in der der betreffende Rand als Ansicht erkennbar ist.

Es ist ohne weiteres ersichtlich, dass auch die Wechselrichterstufe 17 in das Material der Dielektrikumsschicht 5 integriert sein kann, sodass das Steuergerät 2 in diesem Fall lediglich die Gleichspannungsversorgung zu liefern hätte.

Es ist für den Fachmann ferner klar, dass nicht nur die Integration der Hoch-spannungsstufe 36 und ggf. der Wechselrichterstufe 17 in das Material der Dielektrikumsschicht 5 möglich ist, sondern auch die Anordnung der Hochspannungsstufe 36 und ggf. der Wechselrichterstufe 17 mit Mikrobauelementen auf der Dielektrikumsschicht 5 als Träger, wenn auf die Dielektrikumsschicht 5 in diesem Fall eine Abdeckung, vorzugsweise in flexibler Form, aufgebracht wird, um einen Berührungsschutz der Bauelemente zu gewährleisten.

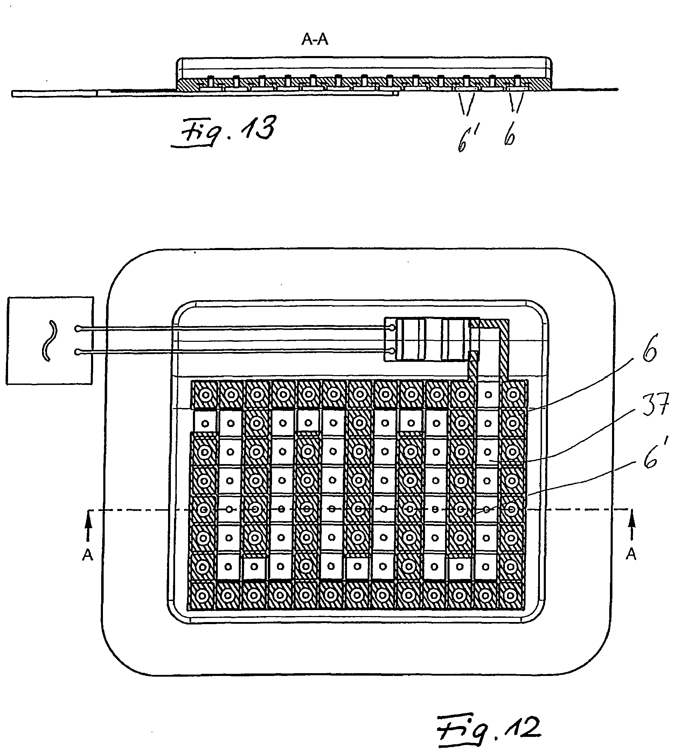

Bei der in den Figuren 12 und 13 dargestellten Modifikation des Auflagestücks sind zwei Elektroden 6, 6' vorgesehen, die durch isolierende Abschnitte 37 der Dielektrikumsschicht 5 voneinander getrennt sind.

Die beiden Elektroden 6, 6' arbeiten vorzugsweise beide als Hochspannungs-elektroden und werden mit gegenpoligen Hochspannungsimpulsen angesteuert. Die Wirkung des Plasmafelds entsteht dadurch, dass beide Elektroden den Kör-per des Patienten als Gegenelektrode benutzen, dass jedoch zwischen den bei-den Elektroden ein verstärktes elektrisches Feld vorhanden ist, das die Plasmabildung verstärkt und begünstigt.

Denkbar ist allerdings auch, die Elektroden 6, 6' so zu schalten, dass die Elektro-de 6' als Gegenelektrode zur Elektrode 6 fungiert, sodass das Plasmafeld nur durch eine Oberflächenentladung zwischen den Elektroden 6, 6' entsteht. Für eine Wundbehandlung ist es allerdings in nahezu allen Fällen sinnvoll, die Haut mit der Wunde des Patienten als Gegenelektrode zu verwenden.

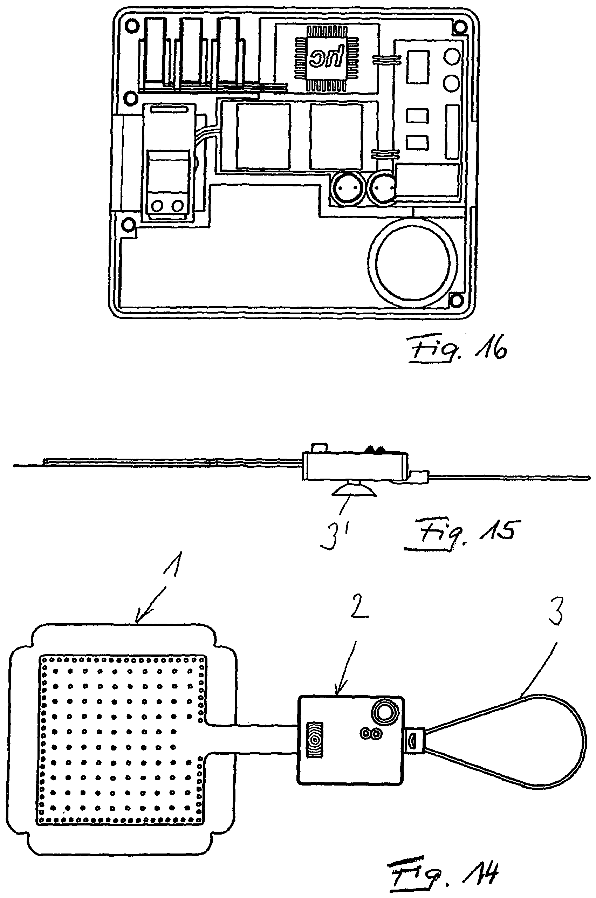

Die in den Figuren 14 bis 16 dargestellte Ausführungsform entspricht der Aus-führungsform gemäß Figur 1, jedoch ohne Absaugpumpe 23. Die Entfernung des Wundsekrets durch die Durchgangsöffnungen 32 erfolgt hierbei ausschließlich durch das saugende Material im Absaugraum 33.

Die beschriebenen Ausführungsformen begrenzen die möglichen Ausbildungen des erfindungsgemäßen Behandlungsgeräts nicht. Insbesondere ist es nicht erforderlich, das Auflagestück 1 unmittelbar über den zungenförmigen Ansatz 11 mit dem Steuergerät zu kontaktieren. Ferner ist es nicht erforderlich, eine lösbare Verbindung zwischen Auflagestück 1 und Steuergerät 2 herzustellen, wenn das Steuergerät 2 nach der Beendigung der Behandlung zusammen mit dem Auflagestück 1 entsorgt wird. In diesem Fall ist eine feste oder klemmende, nicht lösbare Verbindung zwischen Auflagestück 1 und Steuergerät 2 möglich und sinnvoll.

## Patentansprüche

1. Behandlungsgerät für eine dielektrisch behinderte Plasmabehandlung einer Wund- oder Hautfläche, mit einer flexiblen flächigen Elektrodenanordnung mit wenigstens einer flächigen und flexiblen Elektrode (6, 6') und einer flächigen und flexiblen Dielektrikumsschicht (5), die die wenigstens eine Elektrode einbettet und allseitig gegen Berührung abschirmt, und die eine der Wund- oder Hautfläche zugewandte Auflageseite (7) aufweist und die flächige und flexible Elektrode (6, 6') elektrisch von der Wund- oder Hautfläche so abschirmt, dass nur ein dielektrisch behinderter Stromfluss von der Elektrode (6, 6') zur Wund- oder Hautfläche möglich ist, und mit einem ein separates Gehäuse (25) aufweisenden Steuergerät (2), über das die Elektrode (6, 6') mit einer Betriebsspannung verbindbar ist, wobei das Gehäuse (25) des Steuergeräts (2) eine zur Befestigung des Steuergeräts (2) am Körper eines Patienten dienende Befestigungsvorrichtung (3, 3') aufweist, und die Dielektrikumsschicht (5) mit Durchgangsöffnungen (32) versehen ist, **dadurch gekennzeichnet, dass** die Elektrodenanordnung im Bereich der Durchgangsöffnungen (32) mit einer luftdichten Abdeckung (8), die die Flexibilität der Elektrodenanordnung nicht beeinträchtigt, gasdicht abgedichtet überdeckt ist und so einen mit den Durchgangsöffnungen (32) kommunizierenden Fluidraum (33) begrenzt, dass an die Abdeckung (8) eine Leitung (10) angeschlossen ist und dass das Steuergerät (2) eine Pumpe (23) enthält, die über die Leitung (10) mit dem Fluidraum (33) verbindbar ist.

2. Behandlungsgerät nach Anspruch 1, **dadurch gekennzeichnet, dass** der Fluidraum (33) zumindest teilweise mit einem flüssigkeitsabsorbierenden Material gefüllt ist.

3. Behandlungsgerät nach Anspruch 1 oder 2, **dadurch gekennzeichnet, dass** in dem Steuergerät (2) der Pumpe (23) im Strömungsweg ein Behälter (26) zur Aufnahme oder Abgabe von durch die Pumpe (23) transportierter Flüssigkeit vorgeschaltet ist.

4. Behandlungsgerät nach Anspruch 3, **dadurch gekennzeichnet, dass** sich in dem Behälter (26) im Strömungsweg zur Pumpe ein gasdurchlässiger, Flüssigkeit zurückhaltender Filter (28) befindet.

5. Behandlungsgerät nach Anspruch 3 oder 4, **dadurch gekennzeichnet, dass** der Behälter (26) auswechselbar mit der Pumpe (23) und/oder dem Gehäuse (25) verbunden ist.

6. Behandlungsgerät nach einem der Ansprüche 1 bis 5, **dadurch gekennzeichnet, dass** die Anordnung aus Elektrode (6, 6') und Dielektrikumsschicht (5) ein zungenförmiges Anschlussstück (11) ausbildet, das als schmaler Steg in einen Aufnahmeschlitz des Steuergeräts (2) zur Herstellung einer elektrischen Verbindung zwischen der Betriebsspannung und der Elektrode (6, 6') einschiebbar und festlegbar ist.

7. Behandlungsgerät nach einem der Ansprüche 1 bis 6, **dadurch gekennzeichnet, dass** die Verbindung zwischen der Anordnung aus Elektrode (6, 6') und Dielektrikumsschicht (5) und dem Steuergerät (2) mittels einer Bedientaste (12) lösbar ist.

8. Behandlungsgerät nach einem der Ansprüche 1 bis 7, **dadurch gekennzeichnet, dass** die vom Steuergerät (2) der Elektrode (6, 6') zugeführte Betriebsspannung Hochspannungssignale sind.

9. Behandlungsgerät nach einem der Ansprüche 1 bis 8, **dadurch gekennzeichnet, dass** die wenigstens eine Elektrode (6, 6') und die Dielektrikumsschicht (5) in einem einstückigen Auflagestück (1) enthalten sind.

10. Behandlungsgerät nach einem der Ansprüche 1 bis 7 und 9, **dadurch gekennzeichnet, dass** die vom Steuergerät (2) der Elektrode (6, 6') zugeführte Betriebsspannung eine Wechselspannung ist, die auf die Elektrode (6, 6') über eine Hochspannungsstufe (36) gelangt, die auf dem Auflagestück (1) angeordnet ist.

11. Behandlungsgerät nach einem der Ansprüche 1 bis 10, **dadurch gekennzeichnet, dass** das Steuergerät (2) eine Batteriespannungsversorgung aufweist, deren Ausgangsgleichspannung in dem Steuergerät mit einer Wechselrichterstufe (17) in Wechselspannungssignale gewandelt wird.

## Claims

1. Treatment device for a dielectric barrier plasma treatment of a wound surface or skin surface, having a flexible planar electrode arrangement with at least one planar and flexible electrode (6, 6') and a planar and flexible dielectric layer (5) which embeds the at least one electrode and shields it on all sides against touch and which has an application side (7) facing the wound surface or skin surface and electrically shields the planar and flexible electrode (6, 6') from the wound surface or skin surface in such a way that only a dielectric barrier current can flow from the electrode (6, 6') to the wound surface or skin surface, and having a control device (2) which has a separate housing (25) and by means of which the electrode (6, 6') is able to be connected to an operating voltage, wherein the housing (25) of the control device (2) has a fastening apparatus (3, 3') used to fasten the control device (2) to the body of a patient and the dielectric layer (5) is provided with through-openings (32), **characterized in that** the electrode arrangement is covered in a gas-tightly sealed manner with an airtight cover (8), which does not impair the flexibility of the electrode arrangement, in the region of the through-openings (32) and thus delimits a fluid space (33) communicating with the through-openings (32), **in that** a line (10) is connected to the cover (8) and **in that** the control device (2) contains a pump (23) which is able to be connected by means of the line (10) to the fluid space (33).

2. Treatment device according to Claim 1, **characterized in that** the fluid space (33) is at least partially filled with a liquid-absorbent material.

3. Treatment device according to Claim 1 or 2, characterized a container (26) for receiving or releasing liquid transported by the pump (23) is connected upstream of the pump (23) in the flow path in the control device (2) .

4. Treatment device according to Claim 3, **characterized in that** a gas-permeable, liquid-retaining filter (28) is located in the container (26) in the flow path towards the pump.

5. Treatment device according to Claim 3 or 4, **characterized in that** the container (26) is connected interchangeably to the pump (23) and/or the housing (25).

6. Treatment device according to one of Claims 1 to 5, **characterized in that** the arrangement consisting of electrode (6, 6') and dielectric layer (5) forms a tongue-shaped connecting piece (11), which is able to be inserted as a narrow web and fixed in a reception slot of the control device (2) in order to establish an electrical connection between the operating voltage and the electrode (6, 6').

7. Treatment device according to one of Claims 1 to 6, **characterized in that** the connection between the arrangement consisting of electrode (6, 6') and dielectric layer (5) and the control device (2) is able to be released by means of a control button (12).

8. Treatment device according to one of Claims 1 to 7, **characterized in that** the operating voltage supplied by the control device (2) to the electrode (6, 6') is high-voltage signals.

9. Treatment device according to one of Claims 1 to 8, **characterized in that** the at least one electrode (6, 6') and the dielectric layer (5) are contained in a single-piece application piece (1).

10. Treatment device according to one of Claims 1 to 7 and 9, **characterized in that** the operating voltage delivered by the control device (2) to the electrode (6, 6') is an AC voltage, which reaches the electrode (6, 6') via a high-voltage stage (36) which is arranged on the application piece (1).

11. Treatment device according to one of Claims 1 to 10, **characterized in that** the control device (2) has a battery voltage supply, the output DC voltage of which is converted into AC voltage signals in the control device with an inverter stage (17).

## Revendications

1. Appareil de traitement pour le traitement par plasma à barrière diélectrique d'une surface de plaie ou de peau, comprenant un ensemble d'électrodes surfacique flexible avec au moins une électrode surfacique et flexible (6, 6') et une couche diélectrique surfacique et flexible (5) qui enrobe ladite au moins une électrode et qui la protège sur tous les côtés à l'encontre d'un contact, et qui présente une face d'application (7) tournée vers la surface de plaie ou de peau et protège électriquement l'électrode surfacique et flexible (6, 6') vis-à-vis de la surface de plaie ou de peau de telle sorte que seul un courant à barrière diélectrique circulant depuis l'électrode (6, 6') jusqu'à la surface de plaie ou de peau est possible, et comprenant une unité de commande (2) présentant un boîtier (25) séparé, par laquelle l'électrode (6, 6') peut être connectée à une tension de fonctionnement,
dans lequel
le boîtier (25) de l'unité de commande (2) présente un dispositif de fixation (3, 3') qui sert à fixer l'unité de commande (2) au corps d'un patient, et
la couche diélectrique (5) est pourvue d'ouvertures traversantes (32), **caractérisé en ce que**
l'ensemble d'électrodes (6, 6') est recouvert de façon étanche aux gaz dans la zone des ouvertures traversantes (32) d'un recouvrement (8) étanche à l'air qui n'entrave pas la flexibilité de l'ensemble d'électrodes, et délimite ainsi une chambre à fluide (33) communiquant avec les ouvertures traversantes (32) de telle sorte qu'une conduite (10) est raccordée au recouvrement (8) et que l'unité de commande (2) contient une pompe (23) qui peut être reliée à la chambre à fluide (33) par l'intermédiaire de la conduite (10).

2. Appareil de traitement selon la revendication 1,
**caractérisé en ce que** la chambre à fluide (33) est au moins partiellement remplie d'un matériau absorbant les liquides.

3. Appareil de traitement selon la revendication 1 ou 2,
**caractérisé en ce qu'**un récipient (26) destiné à recevoir ou à distribuer un liquide transporté par la pompe (23) est monté dans l'unité de commande (2) en amont de la pompe (23) dans le trajet d'écoulement.

4. Appareil de traitement selon la revendication 3,
**caractérisé en ce qu'**un filtre (28) perméable aux gaz et retenant les liquides est situé dans le récipient (26) dans le trajet d'écoulement vers la pompe.

5. Appareil de traitement selon la revendication 3 ou 4,
**caractérisé en ce que** le récipient (26) est relié de manière interchangeable à la pompe (23) et/ou au boîtier (25).

6. Appareil de traitement selon l'une des revendications 1 à 5,
**caractérisé en ce que** l'ensemble constitué de l'électrode (6, 6') et de la couche diélectrique (5) forme une pièce de raccordement (11) en forme de languette qui peut être insérée et fixée comme une barrette étroite dans une fente de réception de l'unité de commande (2) pour établir une connexion électrique entre la tension de fonctionnement et l'électrode (6, 6').

7. Appareil de traitement selon l'une des revendications 1 à 6,
**caractérisé en ce que** la connexion entre l'ensemble constitué de l'électrode (6, 6') et de la couche diélectrique (5) et l'unité de commande (2) peut être défaite au moyen d'un bouton de commande (12).

8. Appareil de traitement selon l'une des revendications 1 à 7,
**caractérisé en ce que** la tension de fonctionnement fournie de l'unité de commande (2) à l'électrode (6, 6') consiste en signaux à haute tension.

9. Appareil de traitement selon l'une des revendications 1 à 8,
**caractérisé en ce que** ladite au moins une électrode (6, 6') et la couche diélectrique (5) sont contenues dans une pièce d'application (1) d'un seul tenant.

10. Appareil de traitement selon l'une des revendications 1 à 7 et 9,
**caractérisé en ce que** la tension de fonctionnement fournie de l'unité de commande (2) à l'électrode (6, 6') est une tension alternative qui atteint l'électrode (6, 6') par l'intermédiaire d'un étage haute tension (36) disposé sur la pièce d'application (1).

11. Appareil de traitement selon l'une des revendications 1 à 10,
**caractérisé en ce que** l'unité de commande (2) comporte une alimentation en tension de batterie, dont la tension continue de sortie est convertie en signaux de tension alternative dans l'unité de commande par un étage onduleur (17).
